(19) Europäisches Patentamt
European Patent Office
Office européen des brevets

(11) Veröffentlichungsnummer: **0 057 861**
**B1**

(12) # EUROPÄISCHE PATENTSCHRIFT

(45) Veröffentlichungstag der Patentschrift: **10.04.85**

(51) Int. Cl.⁴: **C 07 C 85/04**, C 07 C 87/60, C 07 C 101/58, C 07 C 121/78

(21) Anmeldenummer: **82100577.4**

(22) Anmeldetag: **28.01.82**

(54) **Verfahren zur Herstellung von Nitroanilinen.**

(30) Priorität: **07.02.81 DE 3104310**

(43) Veröffentlichungstag der Anmeldung: **18.08.82 Patentblatt 82/33**

(45) Bekanntmachung des Hinweises auf die Patenterteilung: **10.04.85 Patentblatt 85/15**

(84) Benannte Vertragsstaaten: **CH DE FR GB IT LI**

(56) Entgegenhaltungen:
**DE - A - 2 161 879**
**US - A - 2 305 573**

**Chemical Abstracts, Band 94, Nr. 21, 25. Mai 1981, Columbus, Ohio, USA, NIPPON KAYAKU CO. LTD., "2-Cyano-4-nitroaniline", Seite 689, abstract no. 174702m**

(73) Patentinhaber: **BAYER AG, Konzernverwaltung RP Patentabteilung, D-5090 Leverkusen 1 Bayerwerk (DE)**

(72) Erfinder: **Krüger, Bruno, Dr., Haferkamp 1, D-5000 Köln 80 (DE)**
Erfinder: **Winkler, Adolf, Dr., Christian-Hess-Strasse 69, D-5090 Leverkusen 1 (DE)**
Erfinder: **Hentze, Günter, Dr., Carl-Leverkus-Strasse 4, D-5068 Odenthal (DE)**

**EP 0 057 861 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Nitroanilinen durch Umsetzung von Chlornitrobenzolen mit Ammoniak bei erhöhter Temperatur und erhöhtem Druck.

Aus Gazz. Chim. Ital. 4, 376 (1874) ist die Herstellung von 5-Chlor-2-nitroanilin durch Umsetzung von 2,4-Dichlornitrobenzol mit alkoholischer Ammoniaklösung bekannt. Diese Arbeitsweise ist jedoch mit erheblichen Mängeln behaftet, weil die im Überschuss eingesetzte Reaktionskomponente Ammoniak und das Lösungsmittel Alkohol miteinander mischbar sind und so nur unter erheblichem Aufwand voneinander und vom Reaktionsprodukt getrennt werden können. Die grosse Flüchtigkeit des Alkohols und seine Mischbarkeit mit Wasser erfordern daneben einen grossen technischen Aufwand zur Reinhaltung der Luft und des Wassers, was die Wirtschaftlichkeit dieses Verfahrens stark beeinträchtigt.

Aus US-P 2305573 ist bekannt, Dinitrochlorbenzol mit wässriger Ammoniaklösung zu Dinitranilin umzusetzen. Überträgt man diese Reaktionsweise auf Dichlorbenzole, werden Gemische verschiedener mono- und disubstituierter Aminobenzole gebildet, die nur aufwendig zu trennen sind.

Weiterhin ist aus Recueil 72, 44 (1953) bekannt, durch Umsetzung von 2,4-Dichlornitrobenzol mit wässrigem Ammoniak 5-Chlor-2-nitroanilin herzustellen. Bei diesem Verfahren entstehen jedoch neben dem gewünschten 5-Chlor-2-nitroanilin noch erhebliche Mengen an 2,4-Diaminonitrobenzol, dessen Abtrennung technisch aufwendig und nur unter Beeinträchtigung der Ausbeute an 5-Chlor-2-nitroanilin durchzuführen ist. Wie eigene Versuche ausserdem zeigten, besitzen solche Reaktionsgemische nur eine beschränkte thermische Stabilität, so dass eine Durchführung dieses Verfahrens in technischem Massstab sicherheitstechnische Probleme aufwirft.

Es wurde nun ein Verfahren zur Herstellung von Nitroanilinen der Formel (I)

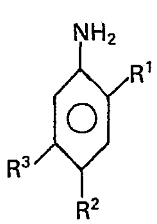

(I),

in der

$R^1$ für Halogen, eine Cyano-, Nitro-, Carboxyl-, Acyl-, Aldehyd- oder eine Sulfogruppe steht,

$R^2$ für Wasserstoff, eine Nitro- oder Carboxylgruppe steht und

$R^3$ Halogen bedeutet, und wobei mindestens einer der Reste $R^1$ oder $R^2$ für eine Nitrogruppe steht,

durch Umsetzung von Chlornitrobenzolen der Formel (II)

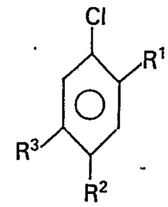

(II),

in der $R^1$, $R^2$ und $R^3$ die oben angegebene Bedeutung haben, mit Ammoniak bei erhöhter Temperatur und erhöhtem Druck gefunden, das dadurch gekennzeichnet ist, dass man die Umsetzung in Gegenwart von chlorierten aromatischen Kohlenwasserstoffen durchführt.

Als Halogene der Formel (I) seien genannt: Fluor, Chlor, Brom, bevorzugt Chlor; als Acylreste seien solche mit 1 bis 6 Kohlenstoffatomen genannt, wie der Acetyl-, Propionyl- und der Benzoylrest, bevorzugt der Acetyl- und der Benzoylrest.

Bevorzugt werden nach dem erfindungsgemässen Verfahren Chlornitrobenzole der Formel (III)

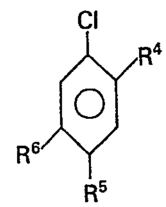

(III),

in der

$R^4$ für Chlor, eine Nitro- oder eine Cyanogruppe steht,

$R^5$ für Wasserstoff, eine Nitro- oder Carboxylgruppe steht und

$R^6$ Wasserstoff oder Chlor bedeutet und wobei mindestens einer der Reste $R^4$ oder $R^5$ für eine Nitrogruppe steht,

umgesetzt.

Besonders bevorzugt wird 2,4-Dichlornitrobenzol in das erfindungsgemässe Verfahren eingesetzt.

Als chlorierte aromatische Kohlenwasserstoffe können solche der Formel (IV)

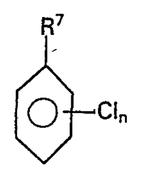

(IV),

worin

$R^7$ einen niederen Alkylrest oder Wasserstoff bedeutet und

n für die Zahl 1, 2 oder 3 steht,

in das erfindungsgemässe Verfahren eingesetzt werden.

Als niedere Alkylreste seien solche mit 1 bis 4, bevorzugt 1 bis 3, Kohlenstoffatomen genannt, wie der Methyl-, der Ethyl-, der n-Propyl-, der iso-Propyl-, der n-Butyl-, der iso-Butyl- und der tert.-Butylrest, bevorzugt der Methyl- und der Ethylrest.

Beispielsweise kommen folgende chlorierte aromatische Kohlenwasserstoffe, die auch im Gemisch untereinander eingesetzt werden können, in Frage: Chlorbenzol, o-Dichlorbenzol, m-Dichlorbenzol, p-Dichlorbenzol, o-, m-, p-Chlortoluol, 1,2,3- und 1,2,4-Trichlorbenzol, bevorzugt Chlorbenzol und o-Dichlorbenzol, besonders bevorzugt Chlorbenzol.

Die Menge der chlorierten aromatischen Kohlenwasserstoffe kann in weiten Bereichen variiert werden. Im allgemeinen bemisst man die Menge der chlorierten aromatischen Kohlenwasserstoffe so, dass mit dem Reaktionsprodukt noch eine gut rührbare Suspension erhalten wird. Üblicherweise beträgt das Gewichtsverhältnis von chlorierten aromatischen Kohlenwasserstoffen zu Einsatzprodukt etwa 1:4 bis 4:1, bevorzugt 1:2 bis 2:1 und besonders bevorzugt 1:1.

Für die erfindungsgemässe Umsetzung genügt es, wenn man pro Mol Ausgangsprodukt 2 Mol Ammoniak einsetzt. Es ist jedoch zweckmässig, zur Aufrechterhaltung eines für den vollständigen Ablauf der Reaktion notwendigen Reaktionsdrucks eine über die Stöchiometrie hinausgehende Menge Ammoniak zuzugeben. Das Molverhältnis von Ausgangsprodukt zu Ammoniak kann dabei etwa 1:3 bis 1:10 betragen. Bevorzugt beträgt das Molverhältnis von Ausgangsprodukt zu Ammoniak 1:4 bis 1:8.

Das erfindungsgemässe Verfahren wird im allgemeinen bei Temperaturen von ca. 100 bis 200°C, vorzugsweise bei 120 bis 160°C durchgeführt. Dabei wird bei einem Überdruck von 10 bis 100 bar, bevorzugt 20 bis 40 bar, gearbeitet.

Die Reaktionszeit der erfindungsgemässen Umsetzung ist u.a. von der Menge der zugegebenen chlorierten aromatischen Kohlenwasserstoffe, dem Ammoniaküberschuss, der Temperatur und dem Druck abhängig und beträgt bei Einhaltung der bevorzugten Werte für die Reaktionsparameter bis zu einem praktisch vollständigen Umsatz ca. 10 bis 25 Stunden.

Das erfindungsgemässe Verfahren kann so durchgeführt werden, dass man das Ausgangsprodukt mit dem chlorierten aromatischen Kohlenwasserstoff vermischt und den Ammoniak bei Raumtemperatur oder Reaktionstemperatur direkt in der gesamten für die Reaktion vorgesehenen Menge oder nur anteilweise zugibt. In einer bevorzugten Ausführungsform wird nur ein Teil des Ammoniaks bei Reaktionstemperatur bis zu einem vorgewählten Reaktionsdruck zugegeben und der Rest des Ammoniaks entsprechend der Abreaktion zudosiert.

Die Aufarbeitung des Reaktionsgemisches und die Isolierung des Reaktionsproduktes kann nach üblichen Verfahren durchgeführt werden. Beim Entspannen des Reaktionsgefässes kann dabei der im Überschuss eingesetzte Ammoniak in einer Absorptionsanlage als wässriger Ammoniak wiedergewonnen oder in einer Druckdestillation als wasserfreier Ammoniak erhalten und wieder in das erfindungsgemässe Verfahren eingesetzt werden.

Das Reaktionsprodukt und die chlorierten aromatischen Kohlenwasserstoffe können aus der zurückbleibenden Produktsuspension durch Filtration oder Destillation voneinander getrennt werden. Eine besonders vorteilhafte Aufarbeitung des Produktgemisches kann durch eine Wasserdampfdestillation bewerkstelligt werden. Dabei gehen kleine Mengen von nicht-umgesetztem Ausgangsprodukt mit dem eingesetzten chlorierten aromatischen Kohlenwasserstoff über. Der wenig Ausgangsprodukt enthaltende chlorierte aromatische Kohlenwasserstoff kann direkt wieder für nachfolgende Ansätze verwendet werden. Aus der erhaltenen wässrigen Produktsuspension kann das Produkt durch Filtration isoliert werden.

Der Vorteil des erfindungsgemässen Verfahrens ist vor allem in der hohen Selektivität mit der die Reaktion bei praktisch vollständigem Umsatz abläuft, zu sehen. Bei der Aminierung von Dihalogennitrobenzolen findet im Gegensatz zum Stand der Technik eine Diaminierung nur in sehr untergeordnetem Masse statt. Auch bei längeren Reaktionszeiten und hohen Ammoniaküberschüssen bleibt die Bildung von Diaminonitrobenzolen vernachlässigbar klein. Dementsprechend sind die erhaltenen Reaktionsgemische thermisch stabil und zeigen keinerlei Neigung zu einer unkontrollierten Zersetzung. Das Reaktionsprodukt fällt in hoher Reinheit an und kann als solches direkt weiterverarbeitet werden. Die Abtrennung der chlorierten aromatischen Kohlenwasserstoffe vom Ammoniak und vom Reaktionsprodukt bereitet keinerlei technische Schwierigkeiten und kann in einfacher Weise durchgeführt werden.

Nitroaniline, insbesondere 5-Chlor-nitroanilin, sind wichtige Vor- und Zwischenprodukte zur Herstellung von Farbstoffen, Pharmazeutika und Pflanzenschutzmitteln (vgl. z.B. DE-OS 2438120, DE-OS 2549417, DE-OS 2363351 und DE-OS 2332343 sowie US-PS 3929821, US-PS 3929822, US-PS 3929824, US-PS 3993769, US-PS 3993768, US-PS 4002640, US-PS 4031234, US-PS 4080461 und US-PS 4034107).

Die nachfolgenden Beispiele sollen das erfindungsgemässe Verfahren verdeutlichen.

Beispiel 1

In einem Edelstahlautoklaven werden bei Raumtemperatur 192 g (1 Mol) 2,4-Dichlornitrobenzol in 400 ml Chlorbenzol mit 200 ml (8 Mol) wasserfreiem Ammoniak versetzt. Der Ansatz wird unter Rühren auf 130°C geheizt und 20 Stunden bei dieser Temperatur gehalten. Der Druck fällt von seinem Höchstwert von 53,5 bar im Laufe der Reaktion auf 36,5 bar. Nach dem Erkalten und Entspannen des Ansatzes wird Chlorbenzol mit Wasserdampf abgetrieben. Die wässrige Produktsuspension wird bei Raumtemperatur abgesaugt und das gelbe kristalline Produkt mit Wasser chloridfrei gewaschen. Es werden nach dem Trocknen 162 g Produkt erhalten, mit einem Gehalt von 95,1%; das entspricht einer Ausbeute von 89,3%.

Wird Ammoniak in 2 Portionen zu je 100 ml eingepumpt und lässt man 2 × je 10 Stunden bei 130°C und Drucken von 34 bis 25 bar und 42 bis 37 bar reagieren, erhält man 172 g einer 93,4%igen Ware entsprechend einer Ausbeute von 93,1%.

Beispiel 2

In einem Edelstahlautoklaven werden 192 g (1 Mol) 2,4-Dichlornitrobenzol mit 200 ml Chlorbenzol auf 140°C erwärmt. 150 ml (6 Mol) wasserfreies Ammoniak werden unter Rühren und Halten der Temperatur bei 140°C so eingepumpt, dass ein Druck von 35 bar nicht überschritten wird. Es werden insgesamt 16 Stunden Einpumpzeit benötigt. Weitere 5 Stunden wird bei 140°C nachreagieren lassen. Darauf wird abgekühlt, entspannt und der Autoklaveninhalt mit insgesamt 1 l Wasser in eine Destillationsapparatur gespült. Hier wird nun Chlorbenzol als Azeotrop mit Wasser abdestilliert und nach Phasentrennung bei dem folgenden Ansatz wieder verwendet. Die wässrige Produktsuspension wird abgesaugt und das gelbe kristalline Produkt bei 60°C im Vakuum getrocknet.

Aus 6 gleichen Ansätzen, bei denen aber immer das zurückgewonnene Chlorbenzol aus dem vorhergehenden Ansatz als Verdünnungsmittel verwendet wird, werden insgesamt 1025 g 96%iges Produkt erhalten, entsprechend einer Ausbeute von 95,1%. Nebenkomponenten: 0,9% 2,4-Dichlornitrobenzol, 1,9% 3-Chlor-4-nitroanilin und 1,0% 2,4-Diaminonitrobenzol. Schmelzpunkt 120 bis 124°C.

Beispiel 3

307 g (1,6 Mol) 2,4-Dichlornitrobenzol werden mit 320 ml o-Dichlorbenzol in einem Edelstahlautoklaven auf 130°C geheizt. Bei dieser Temperatur werden innerhalb von 14 Stunden 240 ml (9,6 Mol) Ammoniak so eingepumpt, dass ein Druck von 35 bar nicht überschritten wird. Es wird 5 Stunden bei 130°C nachgerührt und nach dem Abkühlen und Entspannen der gesamte Ansatz einer Wasserdampfdestillation unterworfen. Nachdem alles o-Dichlorbenzol abgetrieben ist, wird das Produkt durch Filtration isoliert. Es werden 246 g braungelbes Produkt vom Fp. 121 bis 123°C erhalten, mit einem Reingehalt von 95,7%, entsprechend einer Ausbeute von 85,3%.

Beispiel 4

302 g (1,5 Mol) 4-Chlor-3-nitro-benzoesäure werden im Autoklaven in 300 ml Chlorbenzol suspendiert. Bei 120°C werden 225 ml (9 Mol) Ammoniak so eingepumpt, dass ein Druck von 20 bar nicht überschritten wird. 10 Stunden wird bei 120°C nachgerührt. Durch Entspannen und nachfolgende Wasserdampfdestillation wird aufgearbeitet. Man erhält 276,5 g 93%iges Produkt. Das entspricht einer Ausbeute von 94%. Schmp. 250 bis 252°C.

Beispiel 5

Bei 130°C wird in die in einen Autoklaven gerührte Mischung von 182,5 g (1 Mol) 2-Cyan-4-nitrochlorbenzol und 400 ml Chlorbenzol innerhalb 40 Minuten 150 ml (6 Mol) Ammoniak eingepumpt. Der Höchstdruck beträgt 25 bar. Es wird 4 Stunden bei 130°C nachgerührt, dann wie üblich aufgearbeitet. Man erhält 162 g 98%iges Produkt, entsprechend einer Ausbeute von 97%. Der Erstarrungspunkt ist 207,5°C.

Beispiel 6

Im Autoklaven werden 288 g (1,5 Mol) 3,4-Dichlornitrobenzol in 300 ml Chlorbenzol bei 160°C mit 225 ml (9 Mol) Ammoniak umgesetzt. Die Einpumpzeit betrug 9 Stunden; 15 Stunden wurde nachgerührt. Der Druck sank dabei von 57 auf 43 bar.

Ausbeute 243 g Rohprodukt vom Schmp. 101 bis 103°C; GC-Analyse: 93,4%ig ≙ 87,7% der Theorie.

Beispiel 7

In einem 1,3 l-Edelstahlautoklaven wird eine Mischung aus 315 g (2 Mol) 2-Nitrochlorbenzol und 300 ml Chlorbenzol auf 160°C aufgeheizt. Bei dieser Temperatur werden 300 ml (12 Mol) wasserfreies Ammoniak eingepumpt, wobei der Druck bis auf 70 bar ansteigt. Im Verlauf von 20 Stunden bei 160°C sinkt er auf 48 bar. Nach dem Entspannen und der Zugabe von 1000 ml Wasser wird Chlorbenzol zusammen mit Wasser abdestilliert. Es hinterbleibt ein Öl, das beim Abkühlen dunkelgelb kristallin erstarrt. Bei Raumtemperatur wird von der wässrigen Phase abgesaugt und an der Luft getrocknet. Es werden 270 g Rohprodukt vom Schmp. 66 bis 68°C erhalten. Nach GC-Analyse ist das Produkt 93,5%ig, entsprechend einer Ausbeute von 91,5%.

Beispiel 8

202,5 g (1 Mol) 2,4-Dinitrochlorbenzol werden im Autoklaven in 400 ml Chlorbenzol vorgelegt. Bei 100°C werden innerhalb von 4 Stunden 110 ml (4,4 Mol) Ammoniak eingepumpt. Eine weitere Stunde wird bei 100°C nachgerührt, dann durch Entspannen und Wasserdampf destillativ aufgearbeitet. Man erhält 181 g 98%iges Produkt vom Schmp. 177–179°C, entsprechend einer Ausbeute von 97%.

**Patentansprüche**

1. Verfahren zur Herstellung von Nitroanilinen der Formel

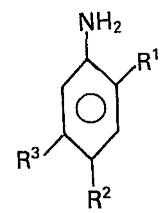

in der

$R^1$ für Halogen, eine Cyano-, Nitro-, Carboxyl-, Acyl-, Aldehyd- oder eine Sulfogruppe steht,

$R^2$ für Wasserstoff, eine Nitro- oder Carboxylgruppe steht und

$R^3$ Halogen bedeutet, und wobei mindestens einer der Reste $R^1$ oder $R^2$ für eine Nitrogruppe steht,

durch Umsetzung von Chlornitrobenzolen der Formel

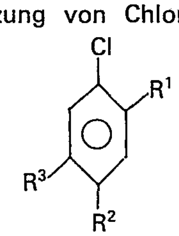

in der
$R^1$, $R^2$ und $R^3$ die oben angegebene Bedeutung haben, mit wasserfreiem Ammoniak bei erhöhter Temperatur und erhöhtem Druck, in Gegenwart von chlorierten aromatischen Kohlenwasserstoffen durchführt.

2. Verfahren nach Anspruch 1, dadurch gekennzeichnet, dass man als chlorierte aromatische Kohlenwasserstoffe solche der Formel

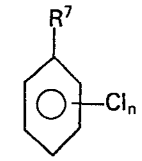

worin
$R^7$ einen niederen Alkylrest oder Wasserstoff bedeutet und
n für die Zahlen 1, 2 oder 3 steht,
einsetzt.

3. Verfahren nach Ansprüchen 1 und 2, dadurch gekennzeichnet, dass man als chlorierte aromatische Kohlenwasserstoffe o-Dichlorbenzol, m-Dichlorbenzol, p-Dichlorbenzol, Chlorbenzol, o-, m-, p-Chlortoluol, 1,2,3- und/oder 1,2,4-Trichlorbenzol einsetzt.

4. Verfahren nach Ansprüchen 1 bis 2, dadurch gekennzeichnet, dass man als chlorierte aromatische Kohlenwasserstoffe Chlorbenzol und/oder o-Dichlorbenzol einsetzt.

5. Verfahren nach Ansprüchen 1 bis 4, dadurch gekennzeichnet, dass man die chlorierten aromatischen Kohlenwasserstoffe und das Einsatzprodukt in einem Gewichtsverhältnis von 1:4 bis 4:1 einsetzt.

6. Verfahren nach Ansprüchen 1 bis 4, dadurch gekennzeichnet, dass man die chlorierten aromatischen Kohlenwasserstoffe und das Einsatzprodukt in einem Gewichtsverhältnis von 1:2 bis 2:1 einsetzt.

7. Verfahren nach Ansprüchen 1 bis 4, dadurch gekennzeichnet, dass man die chlorierten aromatischen Kohlenwasserstoffe und das Einsatzprodukt in einem Gewichtsverhältnis von 1:1 einsetzt.

**Claims**

1. Process for the preparation of nitroanilines of the formula

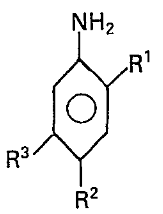

wherein
$R^1$ represents halogen, a cyano group, a nitro group, a carboxyl group, an acyl group, an aldehyde group or a sulpho group,
$R^2$ represents hydrogen, a nitro group or a carboxyl group and
$R^3$ denotes halogen, and wherein at least one of the radicals $R^1$ or $R^2$ represents a nitro group, by the reaction of chloronitrobenzenes of the formula

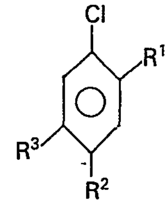

wherein
$R^1$, $R^2$ and $R^3$ have the meaning given above, with anhydrous ammonia, at elevated temperature and under elevated pressure, in the presence of chlorinated aromatic hydrocarbons.

2. Process according to claim 1, characterised in that those hydrocarbons of the formula

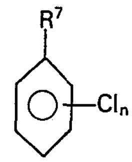

wherein
$R^7$ denotes a lower alkyl radical or hydrogen and
n represents the numbers 1, 2 or 3,
are employed as the chlorinated aromatic hydrocarbons.

3. Process according to claims 1 and 2, characterised in that o-dichlorobenzene, m-dichlorobenzene, p-dichlorobenzene, chlorobenzene, o-, m-, p-chlorotoluene, 1,2,3-trichlorobenzene and/or 1,2,4-trichlorobenzene are employed as the chlorinated aromatic hydrocarbons.

4. Process according to claims 1 to 2, characterised in that chlorobenzene and/or o-dichlorobenzene are employed as the chlorinated aromatic hydrocarbons.

5. Process according to claims 1 to 4, characterised in that the chlorinated aromatic hydrocarbons and the starting material are employed in a ratio by weight of 1:4 to 4:1.

6. Process according to claims 1 to 4, characterised in that the chlorinated aromatic hydrocarbons and the starting material are employed in a ratio by weight of 1:2 to 2:1.

7. Process according to claims 1 to 4, characterised in that the chlorinated aromatic hydrocarbons and the starting material are employed in a ratio by weight of 1:1.

**Revendications**

1. Procédé de préparation de nitranilines de formule

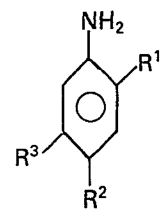

dans laquelle:
$R^1$ représente un halogène, un groupe cyano, nitro, carboxyle, acyle, aldéhyde ou un groupe sulfo,

$R^2$ représente l'hydrogène, un groupe nitro ou carboxyle, et

$R^3$ représente un halogène, l'un au moins des symboles $R^1$ ou $R^2$ représentant un group nitro,

par réaction de chloronitrobenzènes de formule

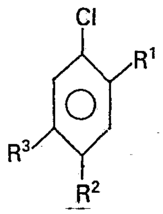

dans laquelle

$R^1$, $R^2$ et $R^3$ ont les significations indiquées ci-dessus, avec l'ammoniac anhydre à température élevée et pression élevée, en présence d'hydrocarbures aromatiques chlorés.

2. Procédé selon la revendication 1, caractérisé en ce que l'on utilise en tant qu'hydrocarbures aromatiques chlorés ceux qui répondent à la formule

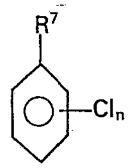

dans laquelle

$R^7$ représente un groupe alkyle inférieur ou l'hydrogène, et

n est égal à 1, 2 ou 3.

3. Procédé selon les revendications 1 et 2, caractérisé en ce que l'on utilise en tant qu'hydrocarbures aromatiques chlorés l'o-dichlorobenzène, le m-dichlorobenzène, le p-dichlorobenzène, le chlorobenzène, l'o-, le m-, le p-chlorotoluène, le 1,2,3- et/ou le 1,2,4-trichlorobenzène.

4. Procédé selon les revendications 1 et 2, caractérisé en ce que l'on utilise en tant qu'hydrocarbures aromatiques chlorés le chlorobenzène et/ou l'o-dichlorobenzène.

5. Procédé selon les revendications 1 à 4, caractérisé en ce que l'on utilise les hydrocarbures aromatiques chlorés et le produit de départ dans des proportions relatives en poids de 1:4 à 4:1.

6. Procédé selon les revendications 1 à 4, caractérisé en ce que l'on utilise les hydrocarbures aromatiques chlorés et le produit de départ dans des proportions relatives en poids de 1:2 à 2:1.

7. Procédé selon les revendications 1 à 4, caractérisé en ce que l'on utilise les hydrocarbures aromatiques chlorés et le produit de départ dans des proportions relatives en poids de 1:1.